# EUROPÄISCHE PATENTSCHRIFT

(11) **EP 2 498 801 B1**
(45) Veröffentlichungstag und Bekanntmachung des Hinweises auf die Patenterteilung: **24.01.2018**
(21) Anmeldenummer: 10776997.8
(22) Anmeldetag: 11.11.2010
(51) Int. Cl.: A61K 9/08, A61K 47/18, A61K 38/26, A61P 3/10

(54) **HARMAZEUTISCHE ZUSAMMENSETZUNG UMFASSEND desPro36Exendin-4(1-39)-Lys6-NH2 UND METHIONIN**
PHARMACEUTICAL COMPOSITION COMPRISING desPro36Exendin-4(1-39)-Lys6-NH2 AND METHIONINE
COMPOSITION PHARMACEUTIQUE COMPRENANT desPro36Exendin-4(1-39)-Lys6-NH2 ET DE LA MÉTHIONINE

(30) Priorität: 13.11.2009 DE 102009052832; 18.03.2010 DE 102010011919
(43) Veröffentlichungstag der Anmeldung: 19.09.2012
(73) Patentinhaber: Sanofi-Aventis Deutschland GmbH, 65929 Frankfurt am Main (DE)
(72) Erfinder: BRUNNER-SCHWARZ, Anette, 65926 Frankfurt (DE); MÜLLER, Werner, 65926Frankfurt am Main (DE); SIEFKE-HENZLER, Verena, 65926 Frankfurt am Main (DE)
(74) Vertreter: Weickmann & Weickmann PartmbB
(86) Internationale Anmeldenummer: PCT/EP2010/067249
(87) Internationale Veröffentlichungsnummer: WO 2011/058082

(56) Entgegenhaltungen:
- WO-A1-2010/044867
- WO-A2-2004/035623
- WO-A2-2005/028516
- WO-A2-2009/102467
- CN-A- 101 366 692
- US-A1- 2001 012 829
- US-A1- 2008 146 490
- US-A1- 2008 260 840

## Beschreibung

Pharmazeutische Zusammensetzung umfassend einen GLP-1-Agonisten und Methionin Gegenstand der vorliegenden Erfindung ist eine flüssige Zusammensetzung umfassend desPro³⁶Exendin-4(1-39)-Lys₆-NH₂ oder/und ein pharmakologisch tolerierbares Salz davon und gegebenenfalls mindestens einen pharmazeutisch akzeptablen Hilfsstoff, dadurch gekennzeichnet, dass sie Methionin enthält und frei von EDTA und Histidin ist und dass die Zusammensetzung desPro³⁶Exendin-4(1-39)-Lys₆-NH₂ in einer Menge von 0,01 mg/ml bis 1,5 mg/ml enthält.

Ein weiterer Gegenstand ist die erfindungsgemäße Zusammensetzung zur Behandlung von Diabetes mellitus. Noch ein weiterer Gegenstand ist die Verwendung einer erfindungsgemäßen Zusammensetzung zur Herstellung eines Arzneimittels zur Behandlung von Diabetes mellitus. Ein weiterer Gegenstand der vorliegenden Anmeldung ist ein Verfahren zur Herstellung einer erfindungsgemäßen Zusammensetzung, umfassend Formulieren eines GLP-1-Agonisten oder/und eines pharmakologisch tolerierbaren Salzes davon mit Methionin und gegebenenfalls mindestens einem pharmazeutisch akzeptablen Hilfsstoff. Noch ein weiterer Gegenstand ist ein Verfahren zur Behandlung eines Patienten mit einer erfindungsgemäßen Zusammensetzung, umfassend Verabreichen der Zusammensetzung an den Patienten.

Übliche Zusammensetzungen von GLP-1-Verbindungen enthalten ein Isotonisierungsmittel, einen Puffer zur Einstellung des pH-Wertes und einen Konservierungsstoff.

WO2001/04156 (Zealand Pharmaceuticals) offenbart eine flüssige Zusammensetzung aus des Ser³⁹-Exendin-4(1-39)-NH₂), Natriumdihydrogenphosphat und Konservierungsmitteln.

WO 2004/035623 (Zealand Pharmaceuticals) offenbart eine flüssige Zusammensetzung umfassend ein stabilisiertes Exendin, 50 mM Histidin, 100 bis 200 mM Saccharose, Mannitol oder einen anderen akzeptablen Zucker, 20 mM Methionin, 20 mM Asparagin-Glutamin oder Asp mit einem pH von 5,3. Die Stabilisierung wird durch bestimmte Modifikationen der Aminosäurebausteine von Exendin-4(1-39) bewirkt, z.B. an den Positionen Gln13, Met14, Trp25 oder Asn28.

WO 2005/021022 (Novo Nordisk) offenbart eine flüssige Zusammensetzung umfassend acetyliertes GLP-1, Phenol als Konservierungsstoff, Mannitol und Glycerin als Isotonisierungsmittel und optional einen Puffer.

WO 2006/051110 (Novo Nordisk) offenbart flüssige Zusammensetzungen, welche Liraglutid (GLP-1-Verbindung), Poloxamer 188 oder Poloxamer 407 (Pluronic F-127) als oberflächenaktive Substanz, Phenol, Propylenglycol und Natriumphosphat (pH 7,7) enthält. Zugabe von Poloxamer-188 oder Poloxamer-407 führten zu einer Stabilisierung.

US 2008/260840 offenbart Suspensionsformulierungen eines insulinotropen Peptids.

US 2008/146490 offenbart eine Vorformulierung, welche eine Mischung mit niedriger Viskosität umfasst, welche eine flüssigkristalline Phase nach Kontakt mit einem wässrigen Fluid bildet. Die Mischung niedriger Viskosität umfasst eine neutrales Diacyllipid und/oder Tocopherol, ein Phospholipid, ein biokompatibles organisches Lösungsmitteln und ein GLP-1-Analog.

US 2001/012829 offenbart Formulierungen umfassend eine stabilisierte GLP-1-Verbindung, an welcher ein lipophiler Substituent befestigt ist.

WO 2005/028516 offenbart Plasmaprotein-Affinitäts-Tags als Wirkstoff-Verabreichungssystem.

WO 2009/102467 offenbart Vorrichtungen, Formulierungen und Verfahren zur Verabreichung von Wirkstoffen.

WO 2010/044867 offenbart hochkonzentrierte Wirkstoffpartikel und Formulierungen und Suspensionen davon.

CN101366692 offenbart eine Exendin-4-Zusammensetzung.

Exendine sind eine Gruppe von Peptiden, welche die Blutglucosekonzentrationen senken können. Exendine weisen eine gewisse Ähnlichkeit der Sequenz zu GLP-1 (7-36) auf (53%, Goke et al. J. Biol Chem 268, 19650-55). Exendin-3 und Exendin-4 stimulieren einen Anstieg der zellulären cAMP-Produktion in Azinuszellen des Meerschweinchenpankreas durch Interaktion mit Exendinrezeptoren (Raufman, 1996, Reg. Peptides 61:1-18). Exendin-3 bewirkt im Gegensatz zu Exendin-4 einen Anstieg der Amylasefreisetzung in Azinuszellen des Pankreas. Exendine wirken als GLP-1-Agonisten.

Glucagon-like peptide 1 (GLP-1) ist ein endokrines Hormon, welches die Insulinantwort nach oraler Aufnahme von Glucose oder Fett erhöht. GLP-1 senkt generell die Glucagonkonzentrationen, verlangsamt die Magenentleerung, stimuliert die (Pro-)Insulin-Biosynthese, erhöht die Empfindlichkeit gegenüber Insulin und stimuliert die Insulin-unabhängige Glycogen-Biosynthese (Holst (1999), Curr. Med. Chem 6:1005, Nauck et al. (1997) Exp Clin Endocrinol Diabetes 105: 187, Lopez-Delgado et al. (1998) Endocrinology 139:2811). Humanes GLP-1 weist 37 Aminosäurereste auf (Heinrich et al., Endocrinol. 115:2176 (1984), Uttenthal et al., J Clin Endocrinol Metabol (1985) 61:472). Aktive Fragmente von GLP-1 schließen GLP-1 (7-36) und GLP-1 (7-37) ein.

Exendin-3, Exendin-4 und Exendinagonisten wurden für die Behandlung von Diabetes mellitus und die Prävention von Hyperglykämie vorgeschlagen, wobei sie die Magenmotilität und -entleerung vermindern (US 5,424,286 und WO98/05351).

Exendin-Analoga können gekennzeichnet sein durch Aminosäuresubstitutionen und/oder C-terminale Trunkierung der nativen Exendin-4-Sequenz. Derartige Exendin-Analoga sind beschrieben in WO 99/07404, WO 99/25727 und WO 99/25728.

Die Festphasensynthese von AVE0010 ist in WO 01/04156 A1 beschrieben. AVE0010 weist die Sequenz: desPro³⁶Exendin-4(1-39)-Lys₆-NH₂ auf. Diese Substanz ist als SEQ ID NO:93 in WO 01/04156 veröffentlicht:

H-G-E-G-T-F-T-S-D-L-S-K-Q-M-E-E-E-A-V-R-L-F-I-E-W-L-K-N-G-G-P-S-S-G-A-P-P-S-
K-K-K-K-K-K-NH2 (SEQ ID NO:1)

Exendin-4 (39 AS) weist die Sequenz auf:

H-G-E-G-T-F-T-S-D-L-S-K-Q-M-E-E-E-A-V-R-L-F-I-E-W-L-K-N-G-G-P-S-S-G-A-P-P-P-
S-NH2 (SEQ ID NO:2)

Exendin-3 weist die Sequenz auf (J. Bio. Chem., 267, 1992, 7402-7405):

H-His-Ser-Asp-Gly-Thr-Phe-Thr-Ser-Asp-Leu-Ser-Lys-Gln-Met-Glu-Glu-Glu-Ala-Val-
Arg-Leu-Phe-Ile-Glu-Trp-Leu-Lys-Asn-Gly-Gly-Pro-Ser-Ser-Gly-Ala-Pro-Pro-Pro-Ser-
NH2 (SEQ ID NO: 3)

GLP-1 weist die Sequenz auf:
H-A-E-G-T-F-T-S-D-V-S-S-Y-L-E-G-Q-A-A-K-E-F-I-A-W-L-V-K-G-R-NH₂ (SEQ ID NO: 4)

Die Aufgabe der vorliegenden Erfindung bestand darin, die Stabilität von flüssigen Formulierungen umfassend AVE0010 zu erhöhen. Insbesondere soll die physikalische und chemische Integrität verbessert werden. Diese Aufgabe wurde dadurch gelöst, dass AVE0010 mit Methionin formuliert wurde.

Es wurde gefunden, dass Methionin die Lagerstabilität einer Zusammensetzung umfassend AVE0010 erhöhen kann. Methionin beeinflusst die physikalische Integrität dieser Zusammensetzungen nicht.

Es wurde überraschenderweise gefunden, dass durch den Zusatz von Methionin die Lagerstabilität einer erfindungsgemäßen Zusammensetzung verbessert werden kann, indem der Anteil der Oxidationsprodukte von Methionin, der Proteine mit hohem Molekulargewicht und der gesamten Verunreinigungen verringert wird. Diese Parameter sind einzeln oder zusammen ein Maß für die chemische Integrität der Zusammensetzungen.

Ferner wurde überraschenderweise gefunden, dass die biologische Aktivität der erfindungsgemäßen Zusammensetzungen durch den Zusatz von Methionin erhöht wurde.

Die Stabilität von pharmazeutisch wirksamen Polypeptiden kann durch verschiedene Mechanismen beeinträchtigt werden. Hierzu gehört der pH, die Temperatur, Licht und die Wirkungen bestimmter Bestandteile.

Eine Reihe üblicher Bestandteile von Formulierungen von GLP-1-Agonisten können nachteilig für die chemische oder/und physikalische Integrität und die Lagerstabilität von Formulierungen sein, welche einen GLP-1-Agonisten enthalten. Dies sind zum Beispiel Polysorbat 20, Polysorbat 80, Poloxamer 188, Benzalkoniumchlorid und Lysin. Die erfindungsgemäßen Zusammensetzungen sind daher bevorzugt frei von diesen Bestandteilen.

Ein Gegenstand der vorliegenden Erfindung ist eine flüssige Zusammensetzung umfassend AVE0010 oder/und ein pharmakologisch tolerierbares Salz davon und gegebenenfalls mindestens einen pharmazeutisch akzeptablen Hilfsstoff, wobei die Zusammensetzung dadurch gekennzeichnet ist, dass sie Methionin enthält.

Die erfindungsgemäße Zusammensetzung enthält Methionin vorzugsweise in einer Menge von 0,5 mg/mL bis 20 mg/mL, stärker bevorzugt in einer Menge von 1 mg/mL bis 5 mg/mL. Methionin kann in der D-Form eingesetzt werden. Ebenso kann Methionin in der L-Form eingesetzt werden. Ebenso können Gemische der D- und der L-Form in beliebigen Proportionen eingesetzt werden.

Insbesondere ist die erfindungsgemäße Zusammensetzung frei von Tensiden, wie Polyolen und Partial- und Fettsäureester und -ether mehrwertiger Alkohole wie des Glycerins und Sorbitols. Die erfindungsgemäßen Zusammensetzungen sind insbesondere frei von Partial- und Fettsäureester und -ether des Glycerins und Sorbitols ausgewählt aus einer Gruppe umfassend Span®, Tween®, Myrj®, Brij®, Cremophor®. Ferner sind die erfindungsgemäßen Zusammensetzungen insbesondere frei von Polyolen, welche ausgewählt werden aus der Gruppe bestehend aus Polypropylenglycolen, Polyethylenglycolen, Poloxameren, Pluronics, Tetronics. Insbesondere ist die erfindungsgemäße Zusammensetzung frei von mindestens einer Substanz ausgewählt aus Polysorbat und Poloxamer.

Insbesondere ist die erfindungsgemäße Zusammensetzung im wesentlichen frei, bevorzugt frei von Polysorbat, wie z. B. Polysorbat 20.

Insbesondere ist die erfindungsgemäße Zusammensetzung im wesentlichen frei, bevorzugt frei von Polysorbat 80.

Insbesondere ist die erfindungsgemäße Zusammensetzung im wesentlichen frei, bevorzugt frei von Poloxamer, wie z. B. Poloxamer 188.
Insbesondere ist die erfindungsgemäße Zusammensetzung im wesentlichen frei, bevorzugt frei von Benzalkoniumchlorid.

Die erfindungsgemäße Zusammensetzung ist frei von Histidin.

Die erfindungsgemäße Zusammensetzung ist frei von EDTA, insbesondere Natrium-EDTA.
Die erfindungsgemäße Zusammensetzung kann eine oder mehrere Substanzen enthalten, welche üblicherweise zur Pufferung des pH-Wertes verwendet werden (Puffersubstanzen). Beispiele derartiger Puffersubstanzen sind Acetat, Citrat und Phosphat z.B. in Mengen von bis zu 5 mg/ml, bis zu 4 mg/ml, bis zu 3 mg/ml oder bis zu 2 mg/ml.
Die erfindungsgemäße Zusammensetzung kann ebenso im wesentlichen frei von Puffersubstanzen sein. Ebenso kann die erfindungsgemäße Zusammensetzung frei von Puffersubstanzen sein.
Die erfindungsgemäße Erfindung kann im wesentlichen frei von Citrat, Acetat und/oder Phosphat sein, oder auch frei von Citrat, Acetat und/oder Phosphat sein.
Insbesondere ist die erfindungsgemäße Zusammensetzung im wesentlichen frei, bevorzugt frei von Histidin und Natrium-EDTA.
Insbesondere enthält die erfindungsgemäße Zusammensetzung kein Insulin.

Die pharmazeutische Zusammensetzung der vorliegenden Erfindung kann einen sauren oder physiologischen pH aufweisen. Ein saurer pH Bereich liegt vorzugsweise im Bereich von pH 1 - 6,8, pH 3,5 - 6,8, oder pH 3,5 - 5. Ein physiologischer pH liegt bevorzugt im Bereich von pH 2,5 -8,5, stärker bevorzugt pH 4,0 bis 8,5, noch stärker bevorzugt pH 6,0 bis 8,5. Besonders bevorzugt ist ein pH-Wert von etwa 4,5. Zur Einstellung des pH-Wertes sind physiologisch unbedenkliche verdünnte Säuren (typischerweise HCl) bzw. Laugen (typischerweise NaOH) geeignet.

Die erfindungsgemäße Zusammensetzung kann ein geeignetes Konservierungsmittel enthalten. Geeignete Konservierungsmittel sind z.B. Phenol, m-Kresol, Benzylalkohol und/oder p-Hydroxybenzoesäureester. Bevorzugt ist m-Kresol.

Ferner kann die erfindungsgemäße Zusammensetzung geeignete Isotonisierungsmittel enthalten. Geeignet sind z.B. Glycerin, Dextrose, Lactose, Sorbitol, Mannitol, Glucose, NaCl, Calcium- oder Magnesium-Verbindungen wie CaCl₂ etc. Die Konzentrationen von Glycerin, Dextrose, Lactose, Sorbitol, Mannitol und Glucose liegen üblicherweise im Bereich von 100 - 250 mM, NaCl in einer Konzentration von bis zu 150 mM. Bevorzugt ist Glycerin.

Insbesondere ist die Zusammensetzung zur parenteralen Verabreichung vorgesehen. Die erfindungsgemäße Zusammensetzung ist vorzugsweise eine injizierbare Zusammensetzung, stärker bevorzugt zur subkutanen Injektion. Insbesondere ist die Zusammensetzung der vorliegenden Erfindung zur Injektion einmal täglich geeignet.

Insbesondere weist die erfindungsgemäße Formulierung nach einer Lagerung von 1 Monat, 2 Monaten, 4 Monaten oder 6 Monaten bei einer Temperatur von +5°C oder 25 °C eine Aktivität von wenigstens 80 %, wenigstens 90 %, wenigstens 95 %, oder wenigstens 98% der Aktivität zum Ausgangszeitpunkt auf.

"Aktivität" bedeutet in der vorliegenden Anmeldung die Aktivität des GLP-1-Agonisten, welcher in der erfindungsgemäßen Formulierung eingesetzt wird. Verfahren zur Bestimmung der Aktivität eines GLP-1-Agonisten sind dem Fachmann bekannt.

Vorzugsweise weist die erfindungsgemäße Zusammensetzung eine biologische Aktivität des GLP-1 -Agonisten von wenigstens 89% oder wenigstens 90% bei Lagerung für 6 Monate bei 25°C auf. Die erfindungsgemäße Zusammensetzung weist vorzugsweise eine biologische Aktivität des GLP-1 -Agonisten von wenigstens 45% oder wenigstens 50% bei Lagerung für 6 Monate bei 40°C auf. Der GLP-1-Agonist ist AVE0010 (Lixisenatid, H-desPro³⁶-Exendin-4-Lys₆-NH₂)

Die erfindungsgemäße Formulierung weist insbesondere nach Lagerung von 1 Monat, 2 Monaten, 3 Monaten, 4 Monaten oder 6 Monaten eine chemische Integrität auf. Chemische Integrität bedeutet insbesondere, dass sie nach einer Lagerung bei einer Temperatur von +5°C, 25°C oder 40°C wenigstens 80 %, wenigstens 90 %, wenigstens 95 %, oder wenigstens 98% des Wirkstoffs zum Ausgangszeitpunkt in im wesentlichen chemisch unveränderter Form enthält.

Chemische Integrität kann die chemische Integrität des GLP-1-Agonisten bedeuten. GLP-1-Agonisten können einen Methioninrest enthalten (z.B. Position 14 in AVE0010). Chemische Integrität des GLP-1-Agonisten bedeutet insbesondere, dass die Oxidation dieses Methioninrestes verhindert wird. Hierbei bedeutet chemische Integrität insbesondere, dass der Anteil an oxidiertem Methionin in Bezug auf das gesamte Methionin im GLP-1 -Agonisten bei Lagerung für 1, 2, 3, 4 oder 6 Monate kleiner als 0,7%, kleiner als 0,6%, kleiner als 0,5 %, kleiner als 0,4% oder kleiner als 0,3 % ist. Die Lagerung kann z.B. bei 5°C, 25°C oder 40°C erfolgen. Bevorzugt ist eine Lagerung für 6 Monate bei 5°C, wobei der Anteil an oxidiertem Methionin kleiner als 0,3% ist. Ebenso bevorzugt ist eine Lagerung für 6 Monate bei 25°C, wobei der Anteil an oxidiertem Methionin kleiner als 0,7%, kleiner als 0,6%, kleiner als 0,5 %, kleiner als 0,4% oder kleiner als 0,3 % ist. ist. Ebenso bevorzugt ist eine Lagerung für 6 Monate bei 40°C, wobei der Anteil an oxidiertem Methionin kleiner als 1 %, kleiner als 0,7%, kleiner als 0,6%, kleiner als 0,5 %, kleiner als 0,4% oder kleiner als 0,3 % ist.

Chemische Integrität kann ein möglichst geringer Anteil an gesamten Verunreinigungen in der erfindungsgemäßen Formulierung bedeuten. Der Anteil der gesamten Verunreinigungen in Bezug auf die Gesamtmasse des in der Formulierung vorhandenen GLP-1 -Agonisten bei Lagerung für 6 Monate bei 40°C ist insbesondere kleiner als 50%, kleiner als 10% bei Lagerung bei 25°C oder/und kleiner als 1,8% bei Lagerung bei 5°C.

Chemische Integrität kann ein möglichst geringer Anteil an Proteinen mit hohem Molekulargewicht in der erfindungsgemäßen Formulierung bedeuten. Der Anteil an Proteinen mit hohem Molekulargewicht in Bezug die Gesamtmasse des in der Formulierung vorhandenen GLP-1 -Agonisten bei Lagerung für 6 Monate bei 40°C ist insbesondere kleiner als 5%, kleiner als 4%, kleiner als 3% oder kleiner als 2%. Bei Lagerung für 6 Monate bei 25°C ist der Anteil an Proteinen mit hohem Molekulargewicht in Bezug auf die Gesamtmasse des in der Formulierung vorhandenen GLP-1 -Agonisten insbesondere kleiner als 0,8%, kleiner als 0,7% oder kleiner als 0,6%.

Die erfindungsgemäße Formulierung weist insbesondere nach Lagerung von 1 Monat, 2 Monaten, 4 Monaten oder 6 Monaten eine physikalische Integrität auf. Physikalische Integrität bedeutet insbesondere, dass sie nach einer Lagerung bei einer Temperatur von +5°C, 25°C oder 40°C wenigstens 80 %, wenigstens 90 %, wenigstens 95 %, oder wenigstens 98% des Wirkstoffs zum Ausgangszeitpunkt in im wesentlichen physikalisch unveränderter Form enthält.

Physikalische Integrität kann die Integrität des GLP-1-Agonisten bedeuten. Physikalische Integrität bedeutet insbesondere, dass der GLP-1-Agonist keine Aggregationen bildet, wie z.B. Fibrillen.

Die Sequenzen von AVE0010 (SEQ ID NO:1), Exendin-4 (SEQ ID NO:2) und Exendin-3 (SEQ ID NO:3) zeigen einen hohen Grad an Übereinstimmung. Die Sequenzen von AVE0010 und Exendin-4 sind in den Positionen 1-37 identisch. Die Sequenz 1-39 aus Exendin-4 ist an 37 der 39 Positionen (94%) identisch mit der Exendin-3-Sequenz an den Positionen 48-86. Anhand der Sequenzen kann der Fachmann hierin angegebene Positionsangaben, welche sich auf eine bestimmte Sequenz beziehen (z.B. auf die Sequenz von AVE0010 oder Exendin-4), ohne weiteres auf andere Sequenzen umrechnen.

Pharmazeutisch tolerierbare Salze können in einem weiteren Verfahrensschritt nach Abschluss der Synthesezyklen des erfindungsgemäßen Verfahrens hergestellt werden. Die Herstellung von pharmazeutisch tolerierbaren Salzen von Peptiden ist dem Fachmann bekannt. Ein bevorzugtes pharmazeutisch tolerierbares Salz ist Acetat.

Ebenso sind pharmakologisch tolerierbare Salze von AVE0010 bevorzugt.

AVE0010 wird in einer Menge von 0,01 mg/ml bis 0,5 mg/ml oder 0,05 mg/ml bis 1,5 mg/ml eingesetzt.

In einer besonderen Ausführungsform umfasst die erfindungsgemäße Formulierung die folgenden Bestandteile:
(a) desPro³⁶Exendin-4(1-39)-Lys₆-NH₂ (z.B. etwa 0,1 mg/mL),
(b) Natriumacetat-Trihydrat (etwa 3,5 mg/mL),
(c) m-Kresol (etwa 2,7 mg/mL),
(d) L-Methionin (etwa 3 mg/mL),
(e) 85%iges Glycerin (etwa 18 mg/mL),
(f) etwa 0,1N Salzsäure, soweit zur Einstellung eines pH-Wertes von etwa 4,5 erforderlich,
(g) etwa 0,1N NaOH-Lösung, soweit zur Einstellung eines pH-Wertes von etwa 4,5 erforderlich, und
(h) Wasser.

Insbesondere besteht die erfindungsgemäße Formulierung aus den genannten Bestandteilen (a) bis (h).

"Etwa" bedeutet in der vorliegenden Anmeldung, dass die Bestandteile z. B. innerhalb der Bereiche von ±10, ±20, oder ±30 um die angegebenen Zahlenwerte in den erfindungsgemäßen Zusammensetzungen vorhanden sein können.

Eine geeignete Verpackung für die erfindungsgemäße Zusammensetzung ist z. B. eine Spritze oder ein Glasgefäß mit einem geeigneten Verschluss, aus denen bei Bedarf einzelne therapeutisch wirksame Dosen entnommen werden können. Ebenso geeignet sind Injektionsstifte ("Stifte", "Pens") zur Verabreichung, welche einen Behälter (z. B. eine Patrone) umfassen, welcher eine erfindungsgemäße pharmazeutische Zusammensetzung enthält.

Offenbart hierin ist ein Verfahren zur Behandlung eines Patienten mit einer erfindungsgemäßen Zusammensetzung, umfassend Verabreichen der Zusammensetzung an den Patienten.

Die erfindungsgemäße Zusammensetzung ist insbesondere zur Verwendung bei der Behandlung von Diabetes mellitus vorgesehen, insbesondere bei der Behandlung von Diabetes mellitus Typ I oder Typ II. Weitere mögliche Indikationen sind Symptome, welche mit Diabetes mellitus assoziiert sind. Bevorzugt wird die erfindungsgemäße Zusammensetzung zur Einstellung der nüchternen, der postprandialen oder/und der postabsorptiven Plasmaglucosekonzentration, zur Verbesserung der Glucosetoleranz, zur Prävention einer Hypoglykämie, zur Prävention eines Funktionsverlustes der β-Zellen des Pankreas, zur Gewichtsabnahme oder/und zur Prävention einer Gewichtszunahme eingesetzt.

Ein weiterer erfindungsgemäßer Gegenstand ist die Verwendung einer erfindungsgemäßen Zusammensetzung zur Herstellung eines Arzneimittels zur Behandlung von Diabetes mellitus, insbesondere von Typ I oder Typ II, oder/und von damit assoziierten Symptomen, wie hierin beschrieben.

Ein weiterer erfindungsgemäßer Gegenstand ist ein Verfahren zur Herstellung einer erfindungsgemäßen Zusammensetzung umfassend Formulieren des AVE0010 GLP-1-Agonisten oder/und eines pharmakologisch tolerierbaren Salzes davon mit Methionin und gegebenenfalls mindestens einem pharmazeutisch akzeptablen Hilfsstoff. Die erfindungsgemäße Zusammensetzung kann zusammen mit der Verabreichung von Metformin, Sulfonylharnstoff oder Glitazonen, einem langwirksamen Insulin/Insulinderivat und/oder einer Kombination davon verwendet werden, insbesondere in einer add-on Therapie zur Verabreichung von Metformin. Die erfindungsgemäße Zusammensetzung kann in Patienten verwendet werden, deren Blutzucker nicht hinreichend durch die Gabe von Metformin, Sulfonylharnstoff oder Glitazonen, einem langwirksamen Insulin/Insulinderivat und/oder einer Kombination davon, kontrolliert bzw. eingestellt werden kann. Die erfindungsgemäße Zusammensetzung kann in Patienten mit Diabetes Typ II als Zusatz zu einer Diät verwendet werden, um die Blutzuckereinstellung/Kontrolle zu verbessern.

Insbesondere enthält die Zusammensetzung des Pro³⁶Exendin-4(1-39)-Lys₆-NH₂ (AVE0010) Liraglutide und/oder ein pharmakologisch tolerierbares Salz zusammen mit Methionin und/oder ein pharmakologisch tolerierbares Salz.

Als langwirksames Insulinderivat wird insbesondere Lantus®, N^{εB29}-tetradecanoyl des (B30) Humaninsulin oder Insuman® genannt.

Besonders bevorzugt ist die erfindungsgemäße Zusammensetzung zur Verwendung bei einer add-on Therapie mit Metformin und/oder einem langwirksamen Insulin/Insulinderivat und/oder einem pharmakologisch tolerierbaren Salz davon zur Behandlung von Diabetes Typ II und/oder Obesity, insbesondere von Patienten, die jünger sind als 50 Jahre und/oder einen Bodymaß Index von mindestens 30 haben. Die erfindungsgemäße Zusammensetzung ist insbesondere zur Verwendung bei einer add-on Therapie für die Behandlung von Diabetes Typ II mit Metformin und AVE0010. Metformin und AVE0010 können verabreicht werden in einem Zeitintervall von 24 h. Metformin und AVE0010 können jeweils verabreicht werden in einer one-a-day Dosierung. Metformin und AVE0010 können verabreicht werden mittels verschiedener Verabreichungswege. Metformin kann oral verabreicht werden, AVE0010 subkutan.

Die Patienten, die mit der erfindungsgemäßen Zusammensetzung behandelt werden, können einen HbA1c Wert von 7 % bis 10 % haben. Sie haben bevorzugt ein Alter von 18 bis 50 Jahren.

Die erfindungsgemäße Zusammensetzung zur Verwendung bei einer add-on Therapie ist insbesondere anwendbar an Patienten, bei denen Diabetes Typ II nicht hinreichend mit Metformin alleine kontrolliert werden kann.

Insbesondere wird Metformin mindestens 1,0 g/Tag, bevorzugt mindestens 1,5 g/Tag für 3 Monate verabreicht.

Die Erfindung wird weiterhin durch die folgenden Beispiele und Figuren erläutert.

### Legenden

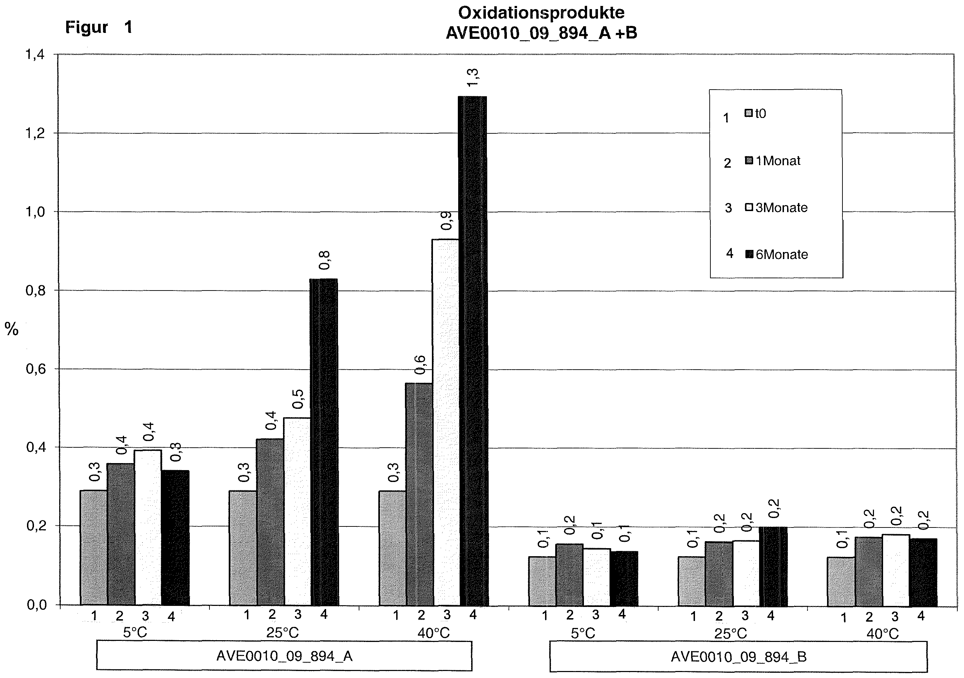

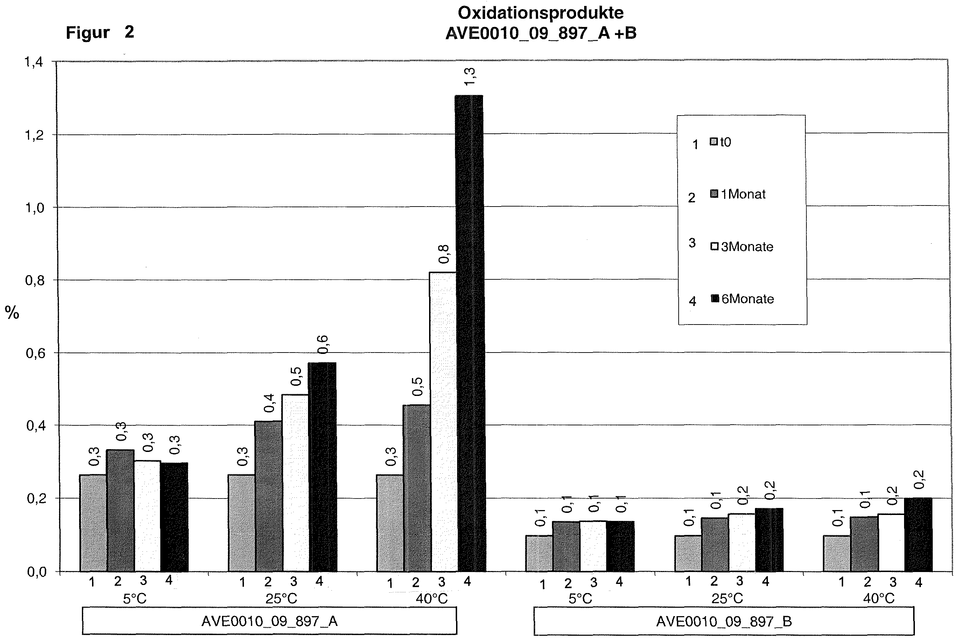

Die Figuren 1 und 2 zeigen den prozentualen Gehalt an oxidiertem Methionin Met(ox) in Bezug auf das gesamte in AVE0010 vorhandene Methionin nach Lagerung bei unterschiedlichen Temperaturen. 1: Ausgangszeitpunkt t0. 2: Lagerung für 1 Monat. 3: Lagerung für 3 Monate. 3: Lagerung für 6 Monate. Figur 1: Ansatz 894. Figur 2: Ansatz 897.

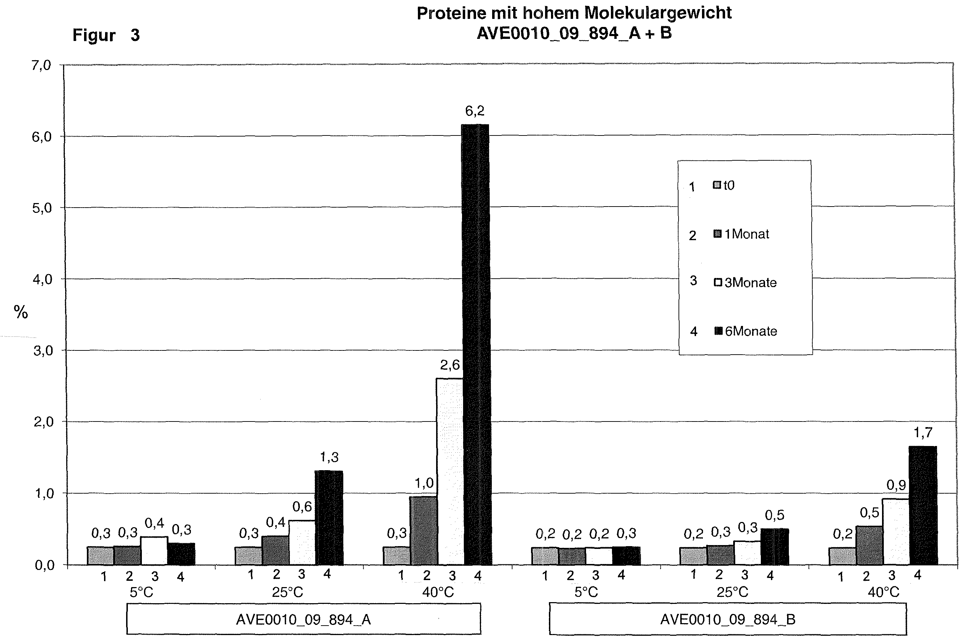

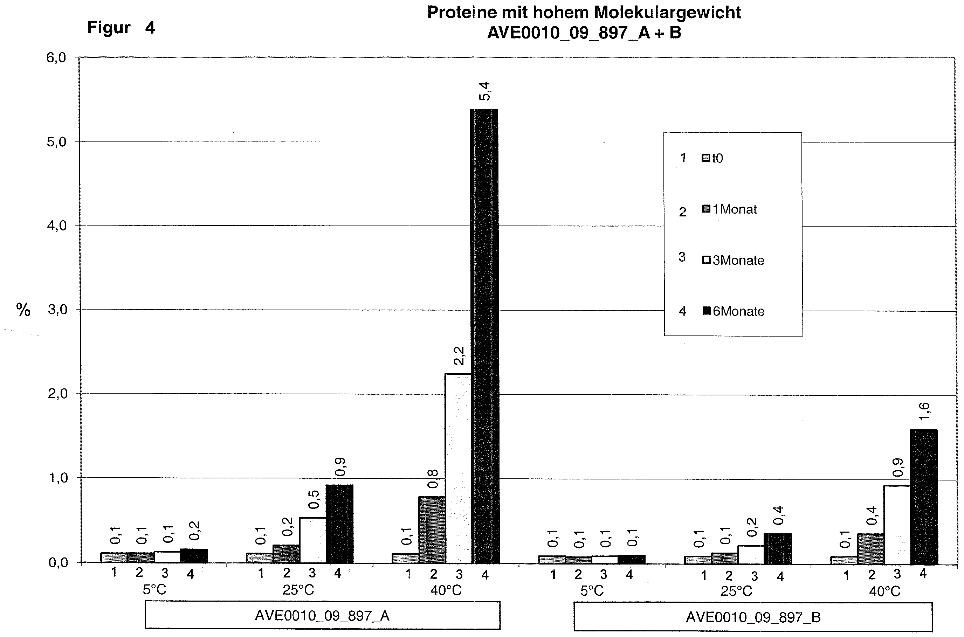

Die Figuren 3 und 4 zeigen den prozentualen Gehalt an Proteinverunreinigungen mit hohem Molekulargewicht (in Bezug auf AVE0010) nach Lagerung bei unterschiedlichen Temperaturen. 1: Ausgangszeitpunkt t0. 2: Lagerung für 1 Monat. 3: Lagerung für 3 Monate. 3: Lagerung für 6 Monate. Figur 3: Ansatz 894. Figur 4: Ansatz 897.

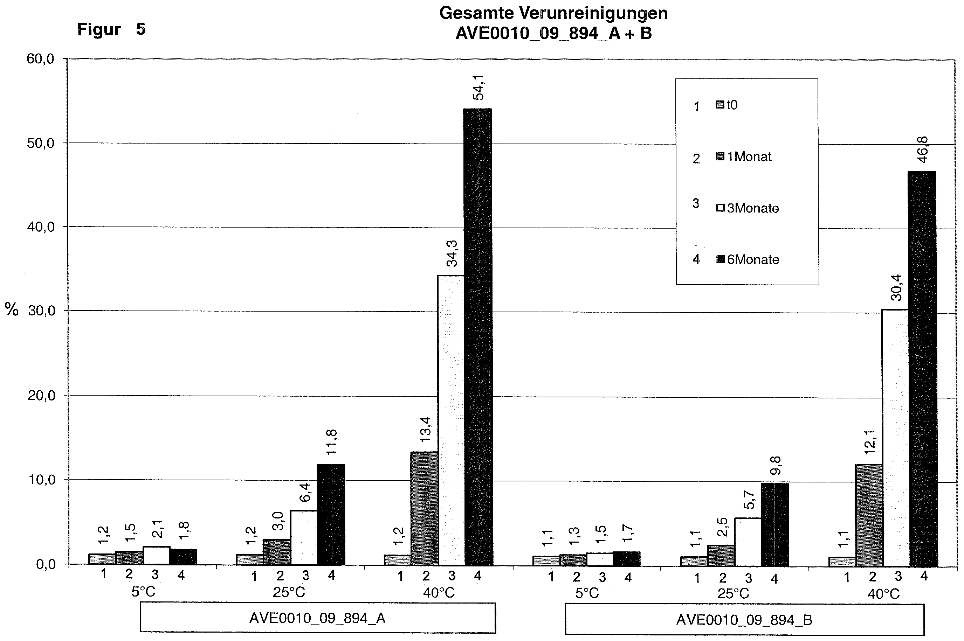

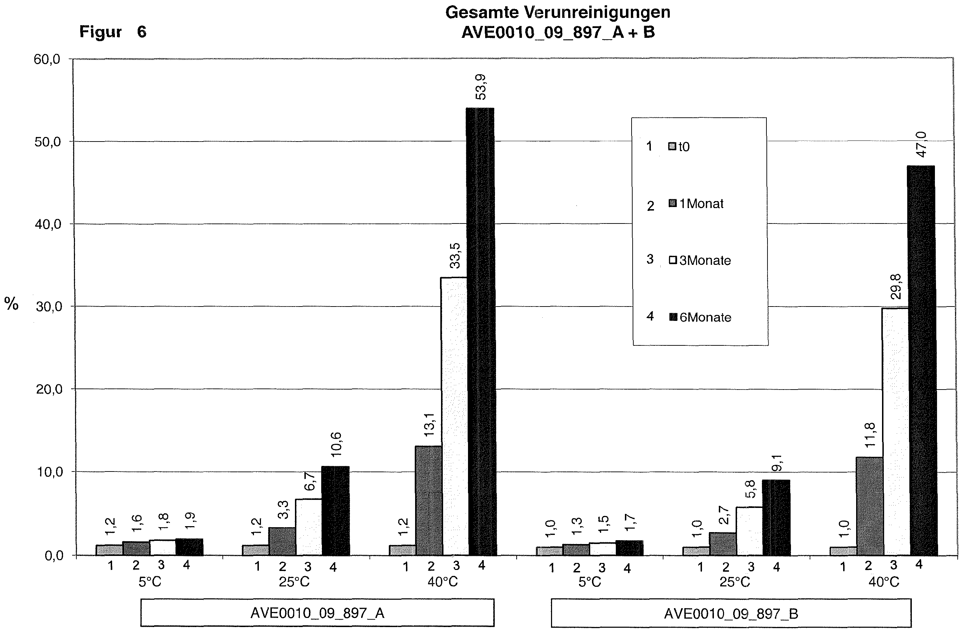

Die Figuren 5 und 6 zeigen den prozentualen Gesamtgehalt an Verunreinigungen (in Bezug auf AVE0010) nach Lagerung bei unterschiedlichen Temperaturen. 1: Ausgangszeitpunkt t0. 2: Lagerung für 1 Monat. 3: Lagerung für 3 Monate. 3: Lagerung für 6 Monate. Figur 5: Ansatz 894. Figur 6: Ansatz 897.

### Beispiel 1

### Flüssige Zusammensetzung umfassend AVE0010 und Methionin

Ziel der Untersuchung ist die Beurteilung der chemischen oder/und physikalischen Stabilität von Formulierungen von AVE0010 (Lösung zur Injektion, 0,1 mg/ml) mit und ohne Methionin, wenn das Produkt in Patronen unter Langzeitbedingungen und beschleunigten Bedingungen für bis zu 6 Monate gelagert wird.

### Folgende Zusammensetzungen werden getestet:

Zusammensetzung A (2 parallele Ansätze AVE0010_09_894_A und AVE0010_09_897_A)

| Substanz | Spezifikation gemäß Arzneibuch | Menge pro Einheit |
|---|---|---|
| AVE0010 | Sanofi-Aventis | 0,10 mg |
| Natriumacetat-Trihydrat | Ph.Eur./USP | 3,50 mg |
| m-Kresol | Ph.Eur./USP | 2,70 mg |
| 85%iges Glycerin | Ph.Eur./USP | 18,00 mg |
| 0,1N Salzsäure | Ph.Eur./USP | ad pH 4,5 |
| 0,1N NaOH-Lösung | Ph.Eur./USP | ad pH 4,5 |
| Wasser zur Injektion (Wfl) | Ph.Eur./USP | ad 1,0 ml |

Zusammensetzung B (2 parallele Ansätze AVE0010_09_894_B und AVE0010_09_897_B)

| Substanz | Spezifikation gemäß Arzneibuch | Menge pro Einheit |
|---|---|---|
| AVE0010 | Sanofi-Aventis | 0,10 mg |
| Natriumacetat-Trihydrat | Ph.Eur./USP | 3,50 mg |
| m-Kresol | Ph.Eur./USP | 2,70 mg |
| L-Methionin | Ph.Eur./USP | 3,00 mg |
| 85%iges Glycerin | Ph.Eur./USP | 18,00 mg |
| 0,1N Salzsäure | Ph.Eur./USP | ad pH 4,5 |
| 0,1N NaOH-Lösung | Ph.Eur./USP | ad pH 4,5 |
| Wasser zur Injektion (Wfl) | Ph.Eur./USP | ad 1,0 ml |

Die Formulierungen werden in Einheiten gelagert, welche für klinische Studien und zum Vertrieb vorgesehen sind

| Bezeichnung | Beschreibung |
|---|---|
| Injektionskartusche | Kartusche, 3 ml farblos, Typ I-Glas (Ph.Eur.), SAP-Nr. 100922 |
| Bördeldeckel und darin eingelegt eine graue Dichtungsscheibe | 7,5 mm |
| | Bördeldeckel: Aluminium |
| | Dichtungsscheibe (außen): Isoprengummi, Mat. Nr. 7773/35 |
| | Dichtungsscheibe (innen): Brombutylgummi, , Mat. Nr. 4780/40 |
| | Typ I-Verschluß (Ph. Eur./USP) |
| | SAP-Nr. 164571 |
| Kolbenstopfen | 9,2 x 11 mm |
| | Brombutylgummi, schwarz |
| | SAP-Nr. 120521 |

Lagerzeiten, Lagerbedingungen, Testzeitpunkte sind in der folgenden Tabelle zusammengefasst.

| Bedingung | Testintervalle (Monate) | | | |
|---|---|---|---|---|
| | 0 | 1 | 3 | 6 |
| Langzeitlagerung | | | | |
| +5 ± 3°C | x | x | x | x |
| Beschleunigte Bedingungen (Temperatur, Luftfeuchtigkeit) | | | | |
| +25 ± 2°C/60 ± 5% RH | | x | x | x |
| +40 ± 2°C/75 ± 5% RH | | x | x | x |

Die Formulierungen werden liegend gelagert. RH bedeutet relative Luftfeuchte. Testzeitpunkt 0 ist der Ausgangszeitpunkt. Die Messergebnisse zum Zeitpunkt 0 werden als Referenz für alle getesteten Bedingungen verwendet. Während der Tests werden die Proben bei +5 ± 3°C gelagert.

Die physikalische und chemische Stabilität der gelagerten Formulierungen wird anhand der folgenden Tests bestimmt.
- Beschreibung
- Klarheit der Lösung und deren Farbe
- pH
- Chemische Stabilität (Reinheit und Verunreinigungen, bestimmt durch HPLC, insbesondere Anteil der Oxidationsprodukte und der gesamten Verunreinigungen)
- Proteine mit hohem Molekulargewicht, bestimmt durch HPSEC
- Sichtbare Partikel
- biologische Aktivität der Formulierungen

### Ergebnisse

Die Formulierungen wurden getrennt für die parallelen Ansätze (894 und 897) auf folgende Parameter untersucht:
- **Biologische Aktivität von AVE0010.** Bei 5°C und 25°C betrug die Aktivität auch nach 6 Monaten mindestens 96% der Ausgangsaktivität. Die Aktivitäten der erfindungsgemäßen Zusammensetzungen waren größer als die Aktivitäten der Vergleichszusammensetzungen. Bei 40 °C betrug die Aktivität nach 6 Monaten in Abwesenheit von Methionin etwa 43%. In Anwesenheit von Methionin betrug die Aktivität etwa 51 %, war also deutlich größer als in Abwesenheit von Methionin.
- **Oxidationsprodukte.** Die Messungen wurden auf einem HPLC-Gerät (Typ alliance) von Water Systems durchgeführt, wobei die 100%-Peakflächenmethode verwendet wurde. Zur Trennung wurde ein Gradient aus 0,1 % TFA und Acetonitril als mobile Phase und eine C18-Umkehrphasensäure (Jupiter) als feste Phase verwendet. Bei 5 °C betrug der Anteil an oxidiertem Methionin Met(ox) in AVE0010 in Abwesenheit von Methionin 0,3 %. Bei 25 °C betrug der Anteil von 0,6 - 0,8 %, bei 40 °C 1,3 %. Enthielt die Formulierung Methionin, so war der Anteil an oxidiertem Methionin deutlich geringer. Er betrug bei allen getesteten Bedingungen maximal 0,2 %. Bei 25 °C war der Anteil also nur etwa 1/4 bis 1/3 der Gehaltes in Abwesenheit von Methionin, bei 40°C sogar nur etwa 1/6 (siehe Figuren 1 und 2).
- **Proteine mit hohem Molekulargewicht.** Bei 5°C betrug der Anteil zwischen 0,1 und 0,3 % und blieb während der gesamten Lagerzeit im wesentlichen unverändert. Bei 25°C stieg der Anteil in Abwesenheit von Methionin auf 0,9 bzw. 1,3 % an. In Anwesentheit von Methionin betrug der Anteil 0,4 bis 0,5 %, war also weniger als halb so groß. Bei 40°C betrug der Anteil in Abwesenheit von Methionin 5,4 % bzw. 6,2%, während er in Anwesentheit von Methionin nur 1,6 bzw. 1,7 % betrug, also deutlich geringer war (siehe Figuren 3 und 4).
- **Gesamte Verunreinigungen.** Bei 5°C stiegen die gesamten Verunreinigungen über die Lagerzeit von 6 Monaten geringfügig von 1,2 bis 1,8 bzw. 1,9 % an (Abwesenheit von Methionin). War Methionin vorhanden, so war der Anstieg etwas geringer. Bei 25 °C wurde ein Anstieg auf 10,6 % bzw. 11,8 % beobachtet. In Anwesenheit von Methionin lagen die Werte unter 10%. Bei 40°C stieg der Anteil auf bis zu 54 % an (ohne Methionin). War Methionin vorhanden, so betrug der Anteil nur etwa 47 % (siehe Figuren 5 und 6).

Die %-Werte sind Gehaltwerte (%-Werte von Verunreinigungen) der Oxidationsprodukte, der Summe von Verunreinigungen und der höher molekularen Proteine (HMWP).

Alle Werte sind per HPLC bestimmt worden, mit der sogenannten 100 %-Methode. Dabei handelt es sich im Speziellen um eine Reverse-Phase-HPLC (C 18 column), bei der eine Gradienten-Methode der mobilen Phase benutzt wurde:
a) 0,1 % TFA, 15 % ACN und b) 0,1 % TFA, 75 % ACN.
Detektion bei 215 nm (UV).

Die hoch molekularen Proteine (HMWP) sind detektiert worden mit HPSEC, beschrieben in der Europäischen Pharmacopoe 6.0 für injezierbare Insulin

### Präparationen.

Die Daten sind in den folgenden Tabellen zusammengefasst.

| **Mittelwerte AVE0010_09_894_A + B** | | | | | | | | |
|---|---|---|---|---|---|---|---|---|
| | **AVE0010_09_894_A** | | | | **AVE0010_09_894_B** | | | |
| **5°C** | t₀ | 1Mon. | 3Mon. | 6Mon. | t₀ | 1Mon. | 3 Mon. | 6 Mon. |
| Gesamte Verunreinigungen | 1,2 | 11,5 | 2,1 | 1,8 | 1,1 | 1,3 | 1,5 | 1,7 |
| Test AVE0010 | 101,5 | 99,6 | 98,0 | 97,8 | 101,1 | 100,5 | 99,4 | 98,6 |
| Proteine mit hohem Molekulargewicht | 0,3 | 0,3 | 0,4 | 0,3 | 0,2 | 0,2 | 0,2 | 0,3 |
| Oxidationsprodukte | 0,3 | 0,4 | 0,4 | 0,3 | 0,1 | 0,2 | 0,1 | 0,1 |
| | | | | | | | | |
| **25°C** | t₀ | 1Mon. | 3Mon. | 6Mon. | t₀ | 1Mon. | 3 Mon. | 6 Mon. |
| Gesamte Verunreinigungen | 1,2 | 3,0 | 6,4 | 11,8 | 1,1 | 2,5 | 5,7 | 9,8 |
| Test AVE0010 | 101,5 | 97,9 | 94,0 | 88,6 | 101,1 | 98,7 | 94,8 | 90,9 |
| Proteine mit hohem Molekulargewicht | 0,3 | 0,4 | 0,6 | 1,3 | 0,2 | 0,3 | 0,3 | 0,5 |
| Oxidationsprodukte | 0,3 | 0,4 | 0,5 | 0,8 | 0,1 | 0,2 | 0,2 | 0,2 |
| | | | | | | | | |
| **40°C** | t₀ | 1Mon. | 3Mon. | 6Mon. | t₀ | 1Mon. | 3 Mon. | 6 Mon. |
| Gesamte Verunreinigungen | 1,2 | 13,4 | 34,3 | 54,1 | 1,1 | 12,1 | 30,4 | 46,8 |
| Test AVE0010 | 101,5 | 87,1 | 66,6 | 42,5 | 101,1 | 88,8 | 70,8 | 50,9 |
| Proteine mit hohem Molekulargewicht | 0,3 | 1,0 | 2,6 | 6,2 | 0,2 | 0,5 | 0,9 | 1,7 |
| Oxidationsprodukte | 0,3 | 0,6 | 0,9 | 1,3 | 0,1 | 0,2 | 0,2 | 0,2 |

| **Mittelwerte AVE0010_09_897_A + B** | | | | | | | | |
|---|---|---|---|---|---|---|---|---|
| | **AVE0010_09_897_A** | | | | **AVE0010_09_897_B** | | | |
| **5°C** | t₀ | 1Mon. | 3Mon. | 6Mon. | t₀ | 1Mon. | 3 Mon. | 6 Mon. |
| Gesamte Verunreinigungen Test AVE0010 | 1,2 | 1,6 | 1,8 | 1,9 | 1,0 | 1,3 | 1,5 | 1,7 |
| | 99,2 | 98,2 | 97,5 | 96,7 | 99,5 | 99,2 | 98,0 | 97,1 |
| Proteine mit hohem Molekulargewicht | 0,1 | 0,1 | 0,1 | 0,2 | 0,1 | 0,1 | 0,1 | 0,1 |
| Oxidationsprodukte | 0,3 | 0,3 | 0,3 | 0,3 | 0,1 | 0,1 | 0,1 | 0,1 |
| | | | | | | | | |
| **25°C** | t₀ | 1Mon. | 3Mon. | 6Mon. | t₀ | 1Mon. | 3 Mon. | 6 Mon. |
| Gesamte Verunreinigungen | 1,2 | 3,3 | 6,7 | 10,6 | 1,0 | 2,7 | 5,8 | 9,1 |
| Test AVE0010 | 99,2 | 96,6 | 92,8 | 87,4 | 99,5 | 97,8 | 93,6 | 90,0 |
| Proteine mit hohem Molekulargewicht | 0,1 | 0,2 | 0,5 | 0,9 | 0,1 | 0,1 | 0,2 | 0,4 |
| Oxidationsprodukte | 0,3 | 0,4 | 0,5 | 0,6 | 0,1 | 0,1 | 0,2 | 0,2 |
| | | | | | | | | |
| **40°C** | t₀ | 1Mon. | 3Mon. | 6Mon. | t₀ | 1Mon. | 3 Mon. | 6 Mon. |
| Gesamt-Verunreinigungen | 1,2 | 13,1 | 33,5 | 53,9 | 1,0 | 11,8 | 29,8 | 47,0 |
| Test AVE0010 | 99,2 | 86,8 | 66,5 | 42,6 | 99,5 | 88,0 | 70,7 | 51,0 |
| Proteine mit hohem Molekulargewicht | 0,1 | 0,8 | 2,2 | 5,4 | 0,1 | 0,4 | 0,9 | 1,6 |
| Oxidationsprodukte | 0,3 | 0,5 | 0,8 | 1,3 | 0,1 | 0,1 | 0,2 | 0,2 |

### Schlussfolgerung

Der Anteil der Oxidationsprodukte, der Proteine mit hohem Molekulargewicht und der gesamten Verunreinigungen sind einzeln oder zusammen ein Maß für die chemische Integrität der Zusammensetzungen. Aus den oben beschriebenen Ergebnissen mit den Beispielzusammensetzungen folgt, dass die erfindungsgemäßen flüssigen Zusammensetzungen umfassend
- einen GLP-1-Agonisten oder/und ein pharmakologisch tolerierbares Salz davon (insbesondere AVE0010 oder/und ein pharmakologisch tolerierbares Salz davon),
- gegebenenfalls mindestens einen pharmazeutisch akzeptablen Hilfsstoff,
- und Methionin,
eine verbesserte Stabilität oder/und chemischen Integrität aufweisen. Der Anteil an oxidiertem Methionin, an Gesamtverunreinigungen und an Proteinen mit hohem Molekulargewicht ist in den erfindungsgemäßen Zusammensetzungen geringer als in den Vergleichszusammensetzungen. Die erfindungsgemäße Zusammensetzung (Ansätze 894_B und 897_B) und die Vergleichszusammensetzungen (Ansätze 894_A und 897_A) unterscheiden sich durch das Vorhandensein von Methionin. Daher kann die Verbesserung der Stabilität oder/und chemischen Integrität auf den Bestandteil Methionin in den erfindungsgemäßen Zusammensetzungen zurückgeführt werden.

### Beispiel 2

In einem weiteren Versuch wurde untersucht, wie sich Na-EDTA und Histidin in einer erfindungsgemäßen Zusammensetzung auswirken.

### Zusammensetzung B (wie in Beispiel 1)

| Substanz | Spezifikation gemäß Arzneibuch | Menge pro Einheit |
|---|---|---|
| AVE0010 | Sanofi-Aventis | 0,10 mg |
| Natriumacetat-Trihydrat | Ph.Eur./USP | 3,50 mg |
| m-Kresol | Ph.Eur./USP | 2,70 mg |
| L-Methionin | Ph.Eur./USP | 3,00 mg |
| 85%iges Glycerin | Ph.Eur./USP | 18,00 mg |
| 0,1N Salzsäure | Ph.Eur./USP | ad pH 4,5 |
| 0,1N NaOH-Lösung | Ph.Eur./USP | ad pH 4,5 |
| Wasser zur Injektion (Wfl) | Ph.Eur./USP | ad 1,0 ml |

### Zusammensetzung C

| Substanz | Spezifikation gemäß Arzneibuch | Menge pro Einheit |
|---|---|---|
| AVE0010 | Sanofi-Aventis | 0,10 mg |
| Natriumacetat-Trihydrat | Ph.Eur./USP | 3,50 mg |
| EDTA Natrium | Ph.Eur./USP | 1,00 mg |
| m-Kresol | Ph.Eur./USP | 2,70 mg |
| L-Methionin | Ph.Eur./USP | 3,00 mg |
| L-Histidin | Ph.Eur./USP | 3,10 mg |
| 85%iges Glycerin | Ph.Eur./USP | 18,00 mg |
| 0,1N Salzsäure | Ph.Eur./USP | ad pH 4,5 |
| 0,1N NaOH-Lösung | Ph.Eur./USP | ad pH 4,5 |
| Wasser zur Injektion (Wfl) | Ph.Eur./USP | ad 1,0 ml |

Nach einem Standard-Studiendesign wurden Kaninchen mit Zusammensetzung B oder C oder einer Saline über den subkutanen (s.c.) oder intramuskulären (i.m.) Weg behandelt. Jeweils die Hälfte der Kaninchen wurde nach 24 h oder 120 h getötet, um die akuten oder subakuten Wirkungen der Verabreichung histologisch zu bestimmen. Außerdem wurde bestimmt, ob eine Reparatur/Regeneration möglicher Veränderungen stattfindet.

Bei subkutaner Injektion von Zusammensetzung C zeigten die Tiere nach 24 h im Unterschied zur Salinekontrolle eine leichte bis moderate inflammatorische Reaktion im subkutanen Bindegewebe. 120 h nach subkutaner Injektion war eine klare Tendenz zur Reparatur der beobachteten Veränderungen durch eine fibroblastische Reaktion zu beobachten. Damit konnte die Verträglichkeit noch als moderat (anstatt als unverträglich) beurteilt werden.

Bei Zusammensetzung B zeigten die Tiere nach subkutaner Injektion keine oder minimale Unterschiede zur Salinekontrolle (gute Verträglichkeit).

Nach intramuskulärer Injektion von Zusammensetzung C zeigten die Tiere eine muskuläre Nekrose (multifocal oder disseminiert), welche klar von den Salinekontrollen zu unterscheiden war, bei denen nur der Injektionskanal als klar umschriebener nekrotischer Bereich sichtbar war. Nach 120 h wurde bei Zusammensetzung C eine Mineralisierung des nekrotischen muskulären Gewebes beobachtet, welche sogar bei der Sektion der Tiere zu sehen war. Obwohl kleine bzw. fokale Mineralisierungen bei Kaninchen an unterschiedlichen Stellen nicht ungewöhnlich sind, waren die Mineralisierungen nach Injektion von Zusammensetzung C klar mit den nekrotischen Bereichen assoziiert. Damit ist die Reversibilität der durch die Injektion hervorgerufenen Läsionen mehr als fraglich. Aufgrund dieser Befunde wurde die Zusammensetzung C nach intramuskulärer Injektion in Kaninchen als unverträglich beurteilt.

Zusammensetzung B zeigte nach intramuskulärer Injektion eine gute Verträglichkeit (keine oder minimale Unterschiede zur Salinekontrolle).

Aus diesen Daten folgt, dass die Zusammensetzung B im Vergleich zu Zusammensetzung C eine verbesserte Verträglichkeit bei intramuskulärer oder subkutaner Verabreichung aufwies. Die subkutane Injektion ist der bevorzugte Verabreichungsweg für die in dieser Anmeldung beschriebenen Zusammensetzungen umfassend einen GLP-1-Agonisten, insbesondere AVE0010.

Damit können die erfindungsgemäßen Zusammensetzungen, welche einen GLP-1-Agonisten umfassen, insbesondere AVE0010, frei von EDTA oder/und Histidin sein. Ebenso können die erfindungsgemäßen Zusammensetzungen im Wesentlichen frei von EDTA und Histidin sein.

## Patentansprüche

1. Flüssige Zusammensetzung umfassend desPro³⁶Exendin-4(1-39)-Lys₆-NH₂ oder/und ein pharmakologisch tolerierbares Salz davon und gegebenenfalls mindestens einen pharmazeutisch akzeptablen Hilfsstoff, **dadurch gekennzeichnet, dass** sie Methionin enthält und frei von EDTA und Histidin ist und dass die Zusammensetzung desPro³⁶Exendin-4(1-39)-Lys₆-NH₂ in einer Menge von 0,01 mg/ml bis 1,5 mg/ml enthält.

2. Flüssige Zusammensetzung gemäß Anspruch 1, **dadurch gekennzeichnet, dass** sie einen pharmazeutisch akzeptablen Konservierungsstoff enthält, insbesondere m-Kresol.

3. Flüssige Zusammensetzung gemäß Anspruch 1 oder 2, **dadurch gekennzeichnet, dass** sie Glycerin enthält.

4. Flüssige Zusammensetzung gemäß einem der vorhergehenden Ansprüche,
**dadurch gekennzeichnet, dass** sie einen pH-Wert von 3,5 bis 5 aufweist.

5. Flüssige Zusammensetzung gemäß einem der vorhergehenden Ansprüche,
**dadurch gekennzeichnet, dass** sie Methionin in einer Menge von 0,5 mg/mL bis 20 mg/mL enthält, bevorzugt in einer Menge von 1 mg/mL bis 5 mg/mL.

6. Flüssige Zusammensetzung gemäß einem der vorhergehenden Ansprüche,
**dadurch gekennzeichnet, dass** sie die folgenden Bestandteile aufweist:
(a) desPro³⁶Exendin-4(1-39)-Lys₆-NH₂,
(b) Natriumacetat,
(c) m-Kresol,
(d) L-Methionin,
(e) 85%iges Glycerin,
(f) etwa 0,1N Salzsäure, soweit zur Einstellung eines pH-Wertes von etwa 4,5 erforderlich,
(g) etwa 0,1N NaOH-Lösung, soweit zur Einstellung eines pH-Wertes von etwa 4,5 erforderlich, und
(h) Wasser.

7. Zusammensetzung nach einem der vorhergehenden Ansprüche zur Verwendung bei der Behandlung von Diabetes mellitus.

8. Zusammensetzung zur Verwendung nach Anspruch 7 zur Behandlung von Diabetes Typ II.

9. Zusammensetzung zur Verwendung nach Anspruch 7 oder 8, **dadurch gekennzeichnet, dass** diese zusammen mit Metformin, einem Sulfonylharnstoff, einem Glitazone und/oder einem langwirksamen Insulin und/oder einer Kombination davon und/oder einem pharmakologisch tolerierbaren Salz davon verabreicht wird.

10. Zusammensetzung zur Verwendung nach Anspruch 9, **dadurch gekennzeichnet, dass** es sich bei der Verabreichung um eine add-on Therapie in Patienten handelt, die keine hinreichende Blutzuckerkontrolle mit Metformin, einem Sulfonylharnstoff, einem Glitazone und/oder einem langwirksamen Insulin erreichen konnten.

11. Zusammensetzung zur Verwendung gemäß Anspruch 9 oder 10, **dadurch gekennzeichnet, dass** die behandelten Patienten einen HbA1 c Wert von 7 % bis 10 % haben.

12. Zusammensetzung zur Verwendung gemäß einem der Ansprüche 7 bis 11 zur Verabreichung in Patienten einmal täglich.

13. Zusammensetzung gemäß einem der Ansprüche 1 bis 6 zur Verwendung als Zusatz zu einer Diät zur Verabreichung an Patienten mit Diabetes Typ II, um die Blutzuckerkontrolle zu verbessern.

14. Zusammensetzung zur Verwendung gemäß Anspruch 13 zur Verabreichung in Patienten einmal täglich.

## Claims

1. Liquid composition comprising desPro³⁶Exendin-4(1-39)-Lys₆-NH₂ and/or a pharmacologically tolerable salt thereof and optionally at least one pharmaceutically acceptable excipient, **characterized in that** it contains methionine and is free of EDTA and histidine and **in that** the composition contains desPro³⁶Exendin-4(1-39)-Lys₆-NH₂ in an amount of from 0.01 mg/ml to 1.5 mg/ml.

2. Liquid composition according to Claim 1, **characterized in that** it contains a pharmaceutically acceptable preservative, more particularly m-cresol.

3. Liquid composition according to Claim 1 or 2, **characterized in that** it contains glycerol.

4. Liquid composition according to any of the preceding claims, **characterized in that** it has a pH of from 3.5 to 5.

5. Liquid composition according to any of the preceding claims, **characterized in that** it contains methionine in an amount of from 0.5 mg/ml to 20 mg/ml, preferably in an amount of from 1 mg/ml to 5 mg/ml.

6. Liquid composition according to any of the preceding claims, **characterized in that** it comprises the following constituents:
(a) desPro³⁶Exendin-4 (1-39) -Lys₆-NH₂,
(b) sodium acetate,
(c) m-cresol,
(d) L-methionine,
(e) 85% glycerol,
(f) about 0.1 N hydrochloric acid, if required for setting a pH of about 4.5,
(g) about 0.1 N NaOH solution, if required for setting a pH of about 4.5, and
(h) water.

7. Composition according to any of the preceding claims for use in the treatment of diabetes mellitus.

8. Composition for the use according to Claim 7 for the treatment of type II diabetes.

9. Composition for the use according to Claim 7 or 8, **characterized in that** it is administered together with metformin, a sulfonylurea, a glitazone and/or a long-acting insulin and/or a combination thereof and/or a pharmacologically tolerable salt thereof.

10. Composition for the use according to Claim 9, **characterized in that** the administration is an add-on therapy in patients who were unable to achieve adequate blood sugar control with metformin, a sulfonylurea, a glitazone and/or a long-acting insulin.

11. Composition for the use according to Claim 9 or 10, **characterized in that** the patients treated have an HbA1c value of from 7% to 10%.

12. Composition for the use according to any of Claims 7 to 11 for administration in patients once a day.

13. Composition according to any of Claims 1 to 6 for use as additive to a diet for administration to type II diabetes patients in order to improve blood sugar control.

14. Composition for the use according to Claim 13 for administration in patients once a day.

## Revendications

1. Composition liquide comprenant du desPro³⁶Exendine-4(1-39)-Lys₆-NH₂ ou/et un sel pharmacologiquement tolérable de celui-ci et éventuellement au moins un adjuvant pharmaceutiquement acceptable, **caractérisée en ce qu'**elle contient de la méthionine et elle est exempte d'EDTA et d'histidine et **en ce que** la composition contient du desPro³⁶Exendine-4(1-39)-Lys₆-NH₂ en une quantité de 0,01 mg/ml à 1,5 mg/ml.

2. Composition liquide selon la revendication 1, **caractérisée en ce qu'**elle contient un conservateur pharmaceutiquement acceptable, en particulier le m-crésol.

3. Composition liquide selon la revendication 1 ou 2, **caractérisée en ce qu'**elle contient du glycérol.

4. Composition liquide selon l'une quelconque des revendications précédentes, **caractérisée en ce qu'**elle présente un pH de 3,5 à 5.

5. Composition liquide selon l'une quelconque des revendications précédentes, **caractérisée en ce qu'**elle contient de la méthionine en une quantité de 0,5 mg/ml à 20 mg/ml, de préférence en une quantité de 1 mg/ml à 5 mg/ml.

6. Composition liquide selon l'une quelconque des revendications précédentes, **caractérisée en ce qu'**elle présente les constituants suivants :
(a) desPro³⁶Exendine-4 (1-39) -Lys₆-NH₂,
(b) acétate de sodium,
(c) m-crésol,
(d) L-méthionine,
(e) glycérol à 85%,
(f) acide chlorhydrique à environ 0,1 N, si nécessaire pour le réglage d'un pH d'environ 4,5,
(g) solution de NaOH à environ 0,1 N, si nécessaire pour le réglage d'un pH d'environ 4,5, et
(h) de l'eau.

7. Composition selon l'une quelconque des revendications précédentes pour une utilisation lors du traitement du diabète sucré.

8. Composition pour une utilisation selon la revendication 7 pour le traitement du diabète de type II.

9. Composition pour une utilisation selon la revendication 7 ou 8, **caractérisée en ce qu'**elle est administrée conjointement avec de la metformine, une sulfonylurée, une glitazone et/ou une insuline à action prolongée et/ou une combinaison de celles-ci et/ou un sel pharmacologiquement tolérable de celles-ci.

10. Composition pour une utilisation selon la revendication 9, **caractérisée en ce qu'**il s'agit, lors de l'administration, d'une thérapie d'appoint chez des patients qui n'ont pas pu atteindre un contrôle suffisant de la glycémie à l'aide de la metformine, d'une sulfonylurée, d'une glitazone et/ou d'une insuline à action prolongée.

11. Composition pour une utilisation selon la revendication 9 ou 10, **caractérisée en ce que** les patients traités présentent une valeur HbA1c de 7% à 10%.

12. Composition pour une utilisation selon l'une quelconque des revendications 7 à 11 pour une administration une fois par jour aux patients.

13. Composition selon l'une quelconque des revendications 1 à 6 pour une utilisation comme supplément à un régime pour l'administration à des patients souffrant d'un diabète de type II, en vue d'améliorer le contrôle de la glycémie.

14. Composition pour une utilisation selon la revendication 13 pour une administration une fois par jour aux patients.
